# EUROPÄISCHE PATENTSCHRIFT

(11) **EP 2 629 724 B1**
(45) Veröffentlichungstag und Bekanntmachung des Hinweises auf die Patenterteilung: **09.12.2020**
(21) Anmeldenummer: 11767728.6
(22) Anmeldetag: 12.10.2011
(51) Int. Cl.: A61F 13/02

(54) **BANDAGE ZUM ANLEGEN AN EINEN MENSCHLICHEN ODER TIERISCHEN KÖRPER**
BANDAGE FOR APPLYING TO A HUMAN OR ANIMAL BODY
BANDAGE DESTINÉ À ÊTRE POSÉ SUR LE CORPS D'UN HOMME OU D'UN ANIMAL

(30) Priorität: 21.10.2010 DE 102010042772
(43) Veröffentlichungstag der Anmeldung: 28.08.2013
(73) Patentinhaber: Karl Otto Braun GmbH & Co. KG., 67752 Wolfstein (DE)
(72) Erfinder: KLÖPPELS, Michael, 52249 Eschweiler (DE); MAAß, Ulrike, 67659 Kaiserslautern (DE)
(74) Vertreter: DREISS Patentanwälte PartG mbB
(86) Internationale Anmeldenummer: PCT/EP2011/067806
(87) Internationale Veröffentlichungsnummer: WO 2012/052333

(56) Entgegenhaltungen:
- DE-U1-202005 011 921
- GB-A- 190 426 355
- US-A- 1 801 825
- US-A- 3 561 441
- US-A- 3 834 983
- US-A- 4 961 418
- US-A1- 2006 253 058
- US-A1- 2006 286 343

## Beschreibung

Die Erfindung betrifft eine Bandage zum Anlegen an einen menschlichen oder tierischen Körper umfassend als Grundstruktur einen flächigen Bandabschnitt mit einer Längserstreckung aus einem textilen Material mit einer ersten und einer zweiten Seite, wobei auf mindestens einer der beiden Seiten zumindest über einen Teil der Längserstreckung der Grundstruktur eine Polsterschicht vorgesehen ist.

Bei der erfindungsgemäßen Bandage soll es sich insbesondere um eine Kompressionsbandage oder Kompressionsbinde handeln.

Im Stand der Technik sind zweilagige Kompressionsverbandsets bekannt, d. h. ein aus separaten Elementen bestehendes Set, wobei ein Element eine Polsterbinde und das andere Element eine Langzugkompressionsbinde ist. Die Binden werden übereinander angelegt. Nachteilig hierbei ist, dass der Anlegevorgang vergleichsweise lange dauert, da zwei Binden übereinander um den entsprechenden Körperteil angelegt werden müssen und darüber hinaus hierdurch die Gefahr, dass Fehler beim Anlegen passieren, deutlich erhöht ist, da die beiden Binden zueinander in der gewünschten Weise ausgerichtet werden müssen. Die Kompression wird durch das Zusammenspiel der beiden Binden erzielt, wobei auch die Polsterbinde einen Anteil zur Kompression beitragen kann.

Darüber hinaus sind einteilige Kompressionspolsterbinden bekannt, welche aus zwei separaten Materiallagen bestehen, beispielsweise ein Laminat aus einem kohäsiven Vlies und einem Schaumstoffpolster oder ein mit einem Gewirk vernadeltes Vlies.

So ist beispielsweise aus der WO 95/16416 eine Kompressionsbandage bekannt, bei der zwischen zwei Schichten aus einem gestrickten Material eine Abstandschicht aus Fäden vorgesehen ist.

Eine ähnliche Ausgestaltung einer medizinischen Bandage ist aus der EP 0 752 839 B1 bekannt.

Die DE 79 29 812 U1 beschreibt eine medizinische Polsterbinde, die zur Unterpolsterung von Stützverbänden dient. Die Polsterbinde besteht dabei aus einem vollsynthetischen Vlies, das aus zwei Schichten mit unterschiedlichen Faserstärken besteht.

Dabei ist eine gattungsgemäße Bandage aus US 4,961,418 A bekannt. Allerdings handelt es sich nicht um eine Kompressionsbinde oder Bandage im Sinne einer flächigen Struktur, die um ein Körperteil durch Umwickeln angelegt werden kann.

Des Weiteren ist aus der DE 20 2005 011 921 U1 eine medizinische, sport-und veterinärmedizinische Bandage bekannt, bei der eine stabilisierende und isolierende Schicht aus geschlossenzelligem hautfreundlichem Neopren auf beiden Seiten mit jeweils einer Schicht aus textilem Material so kaschiert (verklebt) ist, dass die innere auf der Haut aufliegende Schicht durch die Oberflächengestaltung zwischen Haut und Bandage eine Atmung und eine geregelte Schweiß- und Wärmeabführung ermöglicht, wozu Kanäle in einer Polsterschicht vorgesehen sind.

Ausgehend vom Stand der Technik soll es nun Aufgabe der Erfindung sein, eine medizinische Bandage oder Binde bereitzustellen mit einem Polster, die einfach anzulegen und zu handhaben ist und einen guten Sitz aufweist und darüber hinaus in der Herstellung unaufwendig ist.

Die Erfindung löst diese Aufgabe durch eine Binde oder Bandage, insbesondere eine medizinische Binde oder Bandage mit den Merkmalen des Anspruchs 1.

Hierbei soll es sich insbesondere bei der Bandage oder Binde um eine Kompressionsbinde oder -bandage handeln, die eine integrierte Polsterschicht aufweist. Die Begriffe Binde, Band und Bandage werden im Folgenden synonym verwendet.

Dabei soll die Polsterschicht aus Fäden oder Fasern gebildet sein, die Teil der textilen Grundstruktur sind. Solche Fäden oder Fasern sind insbesondere bei einem Gewebe die Kett- und Schussfäden sowie eingebrachte Polfäden, wobei erfindungsgemäß insbesondere Polgewebe vorgesehen sein können. Darüber hinaus können auch Gewirke als Grundstruktur vorgesehen sein, bei denen Fäden oder Fasern eingebracht oder herausgezogen sind. Schließlich können bei Vliesstoffen, also nicht gewebten Stoffen, Fasern oder Fäden aus der textilen Grundstruktur herausgezogen werden oder ebenfalls eingebracht sein zur Bildung der Polsterschicht. Das Einbringen oder Herausziehen von Fäden oder Fasern aus einer textilen Grundstruktur ist dabei im Stand der Technik grundsätzlich bereits bekannt, beispielsweise in der Teppich- oder Automobiltextilienindustrie, aber auch in der Bekleidungs- und Heimtextilienindustrie. Die eingebrachten oder herausgezogenen Fasern können dabei einen Flor (insbesondere Faserbüschel) oder Schlingen bilden oder ungeordnete Fasern sein.

Die Polsterschicht soll dabei die äußerste, einem Träger der Bandage zugewandte Lage der Bandage bilden.

Dabei bezeichnet Faden ein linienförmiges textiles Flächengebilde, wobei hier zwischen Spinnfasergarnen, hergestellt in Spinnereien, und Filamentgarnen, hergestellt in Chemiefaserbetrieben, unterschieden wird. Fäden können hierbei aus einem oder mehreren Garnen bestehen. Der Begriff Faser beinhaltet sowohl Naturstapelfasern, als auch Chemiestapelfasern, sowie endlose Einzelfilamente.

Durch eine solche Gestaltung einer erfindungsgemäßen Binde oder Bandage wird erreicht, dass diese einfacher in der Handhabung ist, da lediglich eine einzige Bandage angelegt werden muss. Darüber hinaus kann der Sitz der Bandage verbessert werden und Druckstellen können vermieden werden. Binden und Bandagen mit integriertem Polster haben gegenüber Binden und Bandagen, die keine Polsterschicht aufweisen, den Vorteil, dass sie weniger Hautbindenreibeffekte aufweisen und dadurch schonender sind. Hinsichtlich bereits bekannter mehrlagiger Binden und Bandagen bietet die vorliegende Gestaltung eine alternative einfach herzustellende Ausgestaltung. Separat hergestellte Lagen weisen gerade bei individueller Gestaltung in Länge und Breite den Nachteil auf, dass die beiden Lagen nicht passgenau zusammengeführt und verbunden werden können, so dass die Möglichkeiten der individuellen Gestaltung nicht optimal ausgenutzt werden können. Eine Kompressionspolsterbinde mit integrierter Polsterschicht weist gegenüber Binden und Bandagen, die aus zwei separaten Lagen bestehen, aufgrund der individuellen Gestaltungsmöglichkeit der Polsterschicht in Länge und Breite Vorteile bezüglich der Kompressionsdruckverteilung, insbesondere im Kantenbereich zur Vermeidung von Wülsten und Einschnüreffekten auf. Eine aus mehreren separaten Lagen hergestellte Kompressionspolsterbinde birgt zu dem die Gefahr, dass sich schon beim Anlegen, spätestens jedoch beim Bewegen der Extremität die zusammengefügten Lagen, z.B. durch Delamination, wieder von einander lösen und die Kompressionswirkung dadurch herabgesetzt wird.

Besonders bevorzugt ist dabei, dass die Polsterschicht und die textile Grundstruktur unmittelbar benachbart sind.

Die Polsterschicht, die auf mindestens einer Seite der Binde oder Bandage vorgesehen ist, besteht somit nicht aus einer eigenständigen textilen Fläche, die mit dem Grundmaterial in beliebiger Weise, z. B. vernadelt, laminiert, geklebt, vernäht oder auf sonstige Weise verbunden ist, sondern die Polsterschicht bzw. die Polsterschichten bestehen aus Fäden und/oder Fasern, die in das Grundmaterial eingebracht oder aus diesem herausgezogen werden. Die Polsterschicht oder Polsterschichten bilden somit die Fortsetzung der Grundstruktur.

Neben der Anwendung derartiger Binden und Bandagen im Bereich der medizinischen Kompressionsbinden und -bandagen sind auch andere Anwendungsbereiche, wie jede Art von elastischen oder unelastischen Binden, Bandagen und Bändern denkbar.

Dabei kann vorgesehen sein, dass die Polsterschicht über ihre Längserstreckung und/oder Quererstreckung auf der Grundstruktur einheitlich und kontinuierlich ausgebildet ist. Die Längserstreckung der Polsterschicht kann dabei von der Längserstreckung der Grundstruktur abweichen. Darüber hinaus kann auch hinsichtlich der Quererstreckung der Polsterschicht diese von der Quererstreckung der Grundstruktur abweichen. Vorzugsweise stimmen die Längs- und insbesondere die Quererstreckung von Grundstruktur und Polsterschicht jedoch überein.

Besonders bevorzugt ist die Polsterschicht in Höhe, Struktur und/oder bezüglich der eingebrachten und/oder herausgezogenen Fasern und/oder Fäden über ihre Längs- und/oder ihre Quererstreckung variabel oder auch nur bereichsweise vorgesehen. Dabei wird als Höhe der Polsterschicht die Dimension betrachtet, die nicht mit der flächigen Dimension der Grundstruktur zusammenfällt. Die Polsterschicht kann insbesondere in Bereichen als Flor oder Schlingen ausgebildet sein, wobei unter Flor zu verstehen ist, dass lose Faserenden vorgesehen sind, wohingegen bei Schlingen keine losen Faserenden oder Faserbüschel vorgesehen sind, sondern geschlossene Schlaufen aus der Grundstruktur aufragen und die Polsterschicht bilden. Dabei kann die Höhe des Flors bzw. der Schlingen der Polsterschicht über die Längs- oder Quererstreckung der Polsterschicht kontinuierlich oder auch abschnitts- oder musterweise bis hin zu jacquardmäßiger Gestaltung der Höhe variieren. Darüber hinaus kann zwischen Schlingen- und Florgestaltung der Polsterschicht ebenfalls variiert werden, d. h. die beiden können kombiniert sein. Schließlich können verschiedene Fäden oder Fasern eingebracht oder aus der Grundstruktur herausgezogen werden, insbesondere auch verschieden gefärbte Fäden oder Fasern oder verschiedene Materialien oder Faser- oder Fadenstärken, wodurch weitere Gestaltungsmöglichkeiten gegeben sind.

Insbesondere ist es möglich, die Polsterschicht an die jeweilige Nutzung der Binde oder Bandage anzupassen. So kann bei Anwendung der Binde oder Bandage in der Kompressionstherapie eine Binde so konstruiert werden, dass für die Umwicklung des Fußes oder der Ferse eines Beins eine niedrigere Florhöhe vorgesehen ist, damit die Binde oder Bandage nicht zu stark aufträgt und die Flor- bzw. Schlingenhöhe dann in den nachfolgenden Bereichen zunimmt. Etwas Entsprechendes ist auch bei anderen Anwendungen, wie beispielsweise bei Lymphdrainagen und für weitere menschliche und tierische Körperextremitäten denkbar.

Weiterhin sind anatomisch bedingte Auswölbungen wie beispielsweise die Knöchelstruktur des Sprunggelenks oder des Handgelenks durch entsprechende Aussparungen und Anpassungen in der Polsterschicht durch Anpassung der Höhe der Polsterschicht schonender in der Kompressionstherapie zu behandeln und es kann eine höhere Arbeitssicherheit und Anlegesicherheit hierdurch bereitgestellt werden, da Fehlanlegungen durch die vorgegebene Form weitgehend vermieden werden können.

Es ist vorgesehen, dass die Grundstruktur ein gewebtes, gewirktes oder ein nichtgewebtes (Vliesstoff) Material umfasst, insbesondere hieraus besteht. Das Einbringen oder Herausziehen der Fäden und/oder Fasern kann mittels Vernadeln, Tuften, Dilourisieren und/oder Aufrauen erfolgen. Ferner können derartige Binden und Bandagen auch durch das Schneiden der Polfasern und/oder Polfaden-Fäden von doppellagig gewebten Polgeweben erzielt werden, so dass zwei Binden oder Bandagen mit je einer Polsterschicht erhalten werden oder einem Verfahren ähnlich dem Weben von Axminsterteppichen. Dabei werden beim Axminsterweben Faserbüschel während des Webprozesses eingebracht.

Im Bereich des Tuftens gibt es verschiedene Ausführungsformen. Insbesondere können hier Unterschiede in der Faserbüschel- oder Schlingenform, Höhenunterschiede des Flors bzw. der Schlingen sowie Farbmustermöglichkeiten bzw. auch die Möglichkeiten, verschiedene Garnarten und Fäden oder Fasermaterialien zu mischen, bestehen.

Möglich ist hier eine sogenannte Boucléware, auch als Loop bezeichnete Tufting-Schlingenware. Weiter denkbar ist ein Velours; d. h. die Schlingen werden bereits auf der Tufting-Maschine aufgeschnitten und später sauber geschoren. Man erhält eine flache Ware mit gleichmäßigem Oberflächenbild. Neben dem klassischen Velours sind auch Soft- und Semivelours zu nennen. Velours wird auch als Schnittflorware bezeichnet. Möglich sind sowohl Tuftingschlingen als auch Tuftingvelourswaren mit Polnoppenversatz (cross-over loop und cross-over cut). Dabei können in sämtlichen Musterungen sowohl als Boucle als auch als Velours, d. h. als Schlingen, aber auch als Florware, auch Hoch-Tief-Varianten gefertigt werden. Grundsätzlich können auch Mischformen, nämlich Ausgestaltungen mit Velours (Flor) und Schlingencharakter erzielt werden, wobei bereits auf der Tuftingmaschine geschnittene und ungeschnittene Noppen einander abwechseln. Weiterhin kann vorgesehen sein, dass insbesondere auf der der Polsterschicht abgewandten Seite des Grundmaterials eine oder weitere Schichten, gegebenenfalls auch Polsterschichten, angeordnet sind. Diese weiteren Schichten können einen kohäsiven oder adhäsiven Charakter aufweisen. Alternativ können auch Beschichtungen zum Fixieren und Stabilisieren der Grundstruktur, aber auch der Fasern und/oder Fäden für die Polsterschicht vorgesehen sein.

Darüber hinaus ist es durch den Einsatz von Fasern oder Fäden unterschiedlicher Farbe möglich, beispielsweise das Logo des Kunden einzutuften, einzuweben oder mit den übrigen möglichen Verfahren einzubringen.

Weiterhin kann vorgesehen sein, dass eine oder mehrere Lagen der Grundstruktur in Längs- und/oder Querrichtung elastisch gestaltet sind. Dabei kann eine Elastizität insbesondere der Kettfäden der Grundstruktur vorgesehen sein. Die Elastizität kann bei nicht zerstörerischer Kraft-Dehnungs-Prüfung zur Aufnahme einer Hysteresekurve mit einer maximalen Zugkraft von 10 N/cm und einem Kraftaufnehmer von 2,5 kN gemessen insbesondere im Bereich von 3 % bis 240 % liegen, insbesondere aber im Bereich von 40 % bis 180 % liegen. Der mit der Binde/Bandage erzeugbare Kompressionsdruck - als Ruhedruck in vivo am ruhenden, liegenden, menschlichen Bein gemessen mit einem Druckmessgerät der Firma Kikuhime, zwischen angewickelter Binde und der Haut am Übergang von der Achillessehne zum Soleus-Muskel (entspricht dem in der Norm RAL GZ 387 festgelegten Messpunkt B1), wobei zwei Binden in zirkuläre Wicklung mit dabei nach Fertigstellung der Wicklung über dem Drucksensor liegenden vier Lagen überlappend angewickelt werden - soll zwischen 5 mmHg und 150 mmHg liegen, bevorzugt zwischen 10 mmHg und 90 mmHg liegen.

Sofern die Grundstruktur ein gewebtes Material ist, kann es insbesondere in Leinwandbindung gewebt sein. Denkbar sind jedoch auch weitere Bindungsarten, insbesondere können die Bindungsarten auch über die Länge und/oder Breite des Grundmaterials variieren.

Weiterhin ist vorgesehen, dass die Binde oder Bandage auf der nicht mit der Polsterschicht versehenen Seite oder in polsterschichtfreien Bereichen kohäsiv oder adhäsiv ausgebildet oder beschichtet ist. Hiermit sind Vorteile bezüglich der Antirutscheigenschaften und dem verbesserten Halt sowie dem sogenannten Selfgrip verbunden.

Dabei können für die Kettfäden und die Schussfäden des Grundmaterials sowie für die die Polsterschicht bildenden Fäden oder Fasern, insbesondere Polkettfäden, folgende Materialien verwendet werden, wobei Kettfäden bzw. Schussfäden des Grundmaterials und Fäden oder Fasern der Polsterschicht aus den gleichen oder verschiedenen Materialien sein können: Baumwolle, Polyester, Polyamid, Viskose, Polyurethan, Polypropylen, Polyethylen, Polyacryl (Nitril), Polybutylenterephthalat, Polyvinylfluorid, Elasthan, Wolle.

Die Erfindung soll im Folgenden anhand einer Zeichnung näher erläutert werden.

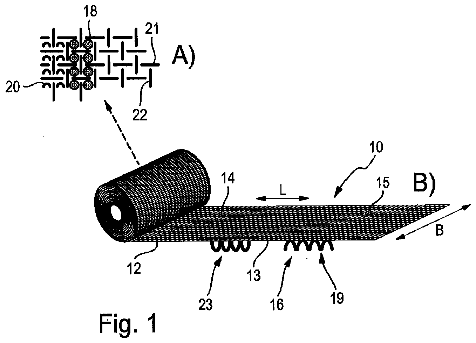

Dabei zeigt die einzige Figur eine mögliche Ausführungsform einer Kompressionspolsterbinde, wobei die Kompressionspolsterbinde in Darstellung B) gezeigt ist. Die Polsterbinde ist in ihrer Gesamtheit mit dem Bezugszeichen 10 bezeichnet und weist eine Längserstreckung auf, die mit dem Doppelpfeil L gekennzeichnet ist, sowie eine Quererstreckung, die mit dem Doppelpfeil B gekennzeichnet ist. Auf einer der beiden Seiten 12 einer Grundstruktur 14, die als flächenhafter Bandabschnitt (13) gestaltet ist und eine sehr viel größere Längserstreckung (L) als Quererstreckung (B) aufweist, ist auf der Seite 12 eine Polsterschicht 16 zumindest partiell über die Längserstreckung der Grundstruktur 14 angebracht. Die weitere Seite der Grundstruktur 14 ist mit 15 bezeichnet. Die Polsterschicht 16 ist dabei durch Fäden oder Fasern gebildet, die in das textile Material der Grundstruktur 14 eingebracht sind. Darstellung A) zeigt die Grundstruktur 14, bei der es sich um ein in Leinwandbindung gewebtes Textil mit Kettfäden 21 und Schussfäden 22 handelt. In das Gewebe sind darüber hinaus Polfäden 18 und 20 eingebracht, wobei diese Polfäden 18 und 20 verschiedene Fadenstärken aufweisen. Die Polfäden 18 weisen freie Fadenenden auf, die aus der Grundstruktur 16 hervorstehen, wie insbesondere Darstellung B) im Bereich 19 entnommen werden kann. Diese Gestaltung wird als Flor oder Velours bezeichnet. Die Polfäden 20 sind dahingegen in Schlingenform ausgebildet, so dass Schlingen oder Loops aus der Grundstruktur 14 hervorstehen (Bereich 23 in Darstellung B). Gemeinsam bilden die Schlingen und der Flor die Polsterschicht 16. Die Polsterschicht 16 wird hierbei insbesondere dadurch erzielt, dass der Faden 18, 20 oder die Fäden, der oder die aus der Grundstruktur 14 herausgezogen oder in die Grundstruktur 14 eingebracht wird bzw. werden, zwischen sich und der Grundstruktur 14 ein Luftpolster bilden und einen geringeren Druck und eine gewisse Nachgiebigkeit beim Anlegen erzielen.

Eine entsprechende Polsterkompressionsbinde 10, wobei die Kettfäden 21 elastisch ausgebildet sind, besitzt den Vorteil einer einfachen Anlegbarkeit bei gleichzeitig herstellungstechnisch günstigen Eigenschaften.

## Patentansprüche

1. Kompressionsbandage zum Anlegen an einen menschlichen oder tierischen Körper umfassend ein flächiges Bandmaterial (13) als Grundstruktur (14) mit einer Längserstreckung (L) aus einem textilen Material mit einer ersten (12) und einer zweiten Seite (15), wobei auf einer der beiden Seiten (12) zumindest über einen Teil der Längserstreckung (L) der Grundstruktur (14) eine Polsterschicht (16) vorgesehen ist, wobei die Polsterschicht (16) aus Fäden und/oder Fasern (18, 20) besteht, die Teil der textilen Grundstruktur (14) sind, nämlich die in die textile Grundstruktur (14) eingebracht und/oder aus dieser herausgezogen sind, **dadurch gekennzeichnet, dass** die Grundstruktur (14) ein gewebtes, gewirktes oder ein nichtgewebtes (Vliesstoff) Material umfasst und die Polsterschicht (16) in Höhe über ihre Längs- und/oder Quererstreckung variiert und eine oder mehrere der Lagen der Grundstruktur (14) in Längs- und/oder Querrichtung elastisch sind, wobei die Polsterschicht durch Anpassung der Höhe der Polsterschicht oder Aussparungen in der Polsterschicht an anatomisch bedingte Auswölbungen angepasst ist und die Bandage auf der nicht mit der Polsterschicht versehenen Seite oder in polsterschichtfreien Bereichen kohäsiv oder adhäsiv ausgebildet oder beschichtet ist.

2. Bandage nach Anspruch 1, **dadurch gekennzeichnet, dass** die Polsterschicht (16) die äußerste einem Träger der Bandage (10) zugewandte Lage der Bandage bildet.

3. Bandage nach Anspruch 1 oder 2, **dadurch gekennzeichnet, dass** die Polsterschicht (16) und die textile Grundstruktur (14) unmittelbar benachbart sind.

4. Bandage nach einem der vorangehenden Ansprüche, **dadurch gekennzeichnet, dass** die Polsterschicht (16) über ihre Längserstreckung auf der Grundstruktur (14) einheitlich ausgebildet ist.

5. Bandage nach einem der vorangehenden Ansprüche, **dadurch gekennzeichnet, dass** die Polsterschicht (16) in Bereichen als Flor (18) und/oder als Schlingen (20) ausgebildet ist.

6. Bandage nach einem der vorangehenden Ansprüche, **dadurch gekennzeichnet, dass** die Grundstruktur (14) aus einem gewebten, gewirkten oder einem nichtgewebten (Vliesstoff) Material besteht.

7. Bandage nach einem der vorangehenden Ansprüche, **dadurch gekennzeichnet, dass** die Grundstruktur ein Polgewebe oder ein mit einem Polfäden oder -fasern einbringenden Verfahren hergestelltes Gewebe oder Gewirke oder Vlies ist.

8. Bandage nach einem der vorangehenden Ansprüche, **dadurch gekennzeichnet, dass** das Einbringen oder Herausziehen der Fäden oder Fasern (18, 20) mittels Vernadeln, Tuften, Dilourisieren oder Aufrauen erfolgt.

9. Bandage nach einem der vorangehenden Ansprüche, **dadurch gekennzeichnet, dass** insbesondere auf der der Polsterschicht (16) abgewandten Seite (15) der Grundstruktur (14) eine oder weitere Schichten angeordnet sind.

## Claims

1. Compression bandage for applying to a human or animal body, comprising a two-dimensional tape material (13) as base structure (14) with a longitudinal extent (L) made of a textile material with a first (12) and a second side (15), wherein on one of the two sides (12) at least over a part of the longitudinal extent (L) of the base structure (14) a cushion layer (16) is provided wherein that the cushion layer (16) is formed by threads and/or fibers (18, 20) which are part of the textile base structure (14), namely which are introduced into the textile base structure (14) and/or are drawn out the base structure (14), **characterized in that** the base structure (14) comprises a woven, knitted or nonwoven (nonwoven) material and the cushion layer (16) varies along its longitudinal and/or transverse extent in height and **in that** one or multiple of the layers of the base structure (14) are elastic in longitudinal and/or transverse direction wherein the cushion layer through adaption of the height or recesses in the cushion layer is adapted to anatomically related protrusions and the bandage is configured cohesive or adhesive on the side not carrying the cushion layer or on cushion layer free areas.

2. Bandage according to claim 1, **characterized in that** the cushion layer (16) forms the outermost layer of the bandage which faces a wearer of the bandage (10).

3. Bandage according to claim 1 or 2, **characterized in that** the cushion layer (16) and the textile base structure (14) are directly adjacent one another.

4. Bandage according to one of the preceding claims, **characterized in that** the cushion layer (16) is configured uniform across its longitudinal extent on the base structure (14).

5. Bandage according to one of the preceding claims, **characterized in that** regions of the cushion layer (16) are configured as pile (18) and/or loops (20).

6. Bandage according to one of the preceding claims, **characterized in that** the base structure (14) is formed by a woven, knitted or nonwoven (nonwoven) material.

7. Bandage according to one of the preceding claims, **characterized in that** the base structure is a pile woven fabric or a woven, knitted or nonwoven fabric which is produced with a method which introduces pile threads or pile fibers.

8. Bandage according to one of the preceding claims, **characterized in that** the introducing or pulling out of the threads or fibers (18, 20) is performed by means of needling, tufting, dilourizing or roughening.

9. Bandage according to one of the preceding claims, **characterized in that** in particular on the side (15) of the base structure (14) which faces away from the cushion layer (16) one or further layers are arranged.

## Revendications

1. Bandage de compression destiné à être placé sur un corps humain ou animal, comprenant comme structure de base (14) un matériau plat en bande (13) avec une étendue longitudinale (L) composé d'un matériau textile avec une première (12) et une deuxième face (15), dans lequel, sur une des deux faces (12), une couche matelassée (16) est prévue au moins sur une partie de l'étendue longitudinale (L) de la structure de base (14), dans lequel la couche matelassée (16) est constituée de fils et/ou fibres (18, 20), qui font partie de la structure de base (14) textile, à savoir qui sont introduits dans la structure de base (14) textile et/ou en sont extraits, **caractérisé en ce que** la structure de base (14) comprend un matériau tissé, tricoté ou non tissé (non-tissé) et la couche matelassée (16) varie en hauteur sur toute son étendue longitudinale et/ou transversale et une ou plusieurs des couches de la structure de base (14) sont élastiques dans la direction longitudinale et/ou transversale, dans lequel la couche matelassée est adaptée à des bombements propres à l'anatomie par adaptation de la hauteur de la couche matelassée ou des évidements dans la couche matelassée et le bandage, sur la face non pourvue de la couche matelassée ou dans des zones exemptes de couche matelassée, est réalisé de manière cohésive ou adhésive ou revêtu.

2. Bandage selon la revendication 1, **caractérisé en ce que** la couche matelassée (16) forme la couche la plus extérieure du bandage, tournée vers un support du bandage (10) .

3. Bandage selon la revendication 1 ou 2, **caractérisé en ce que** la couche matelassée (16) et la structure de base (14) textile sont immédiatement voisines.

4. Bandage selon l'une quelconque des revendications précédentes, **caractérisé en ce que** la couche matelassée (16) est réalisée de manière uniforme sur la structure de base (14) sur toute son étendue longitudinale.

5. Bandage selon l'une quelconque des revendications précédentes, **caractérisé en ce que** la couche matelassée (16) est réalisée dans des zones sous la forme de poil (18) et/ou sous la forme de boucles (20).

6. Bandage selon l'une quelconque des revendications précédentes, **caractérisé en ce que** la structure de base (14) est constituée d'un matériau tissé, tricoté ou non tissé (non-tissé).

7. Bandage selon l'une quelconque des revendications précédentes, **caractérisé en ce que** la structure de base est un tissu à poils ou un tissu ou tricot ou non-tissé fabriqué avec un procédé introduisant des fils ou fibres de poil.

8. Bandage selon l'une quelconque des revendications précédentes, **caractérisé en ce que** l'introduction ou l'extraction des fils ou fibres (18, 20) s'effectue au moyen d'un aiguilletage, touffetage, aiguilletage Dilour ou grattage.

9. Bandage selon l'une quelconque des revendications précédentes, **caractérisé en ce qu'**une ou plusieurs couches sont disposées en particulier sur la face (15) de la structure de base (14) opposée à la couche matelassée (16) .
